# EUROPEAN PATENT APPLICATION

(11) **EP 2 319 493 A2**
(43) Date of publication of application: **11.05.2011**
(21) Application number: 10177243.2
(22) Date of filing: 22.07.2003
(51) Int. Cl.: A61K 9/06, A61K 31/404, A61P 27/02

(54) **Ophthalmic ointment composition comprising a drug, an ointment base and a solubilizing/dispersing agent**

(30) Priority: 23.07.2002 US 397865 P
(62) Divisional of application: 03765094.2
(71) Applicant: Novartis AG, 4056 Basel (CH); Novartis Pharma GmbH, 1230 Wien (AT)
(72) Inventor: Aukunuru, Jithan, Omaha, NE 68105 (US); Babiole Saunier, Maggy, F-68130, Jettingen (FR); Bizec, Jean-Claude, F-68440, Habsheim (FR); Kis, Georg Ludwig, CH-8273, Triboltingen (CH); Schoch, Christian, CH-4132, Muttenz (CH); Wong, Michelle Pik-Han, Duluth, GA 30096 (US)
(74) Representative: Hutchison, John Robert

(57) **Abstract**

This invention relates to a semisolid ophthalmic composition, in particular an ointment, comprising
(1) an ophthalmic drug, e. g. a staurosporine derivative,
(2) an ointment base and
(3) an agent for dispersing and/or dissolving said drug in the ointment base, selected from a poly(ethylene-glycol), a polyethoxylated castor oil, an alcohol having 12 to 20 carbon atoms and a mixture of two or more of said components.

## Description

This invention relates to semisolid ophthalmic compositions comprising an ophthalmic drug, in particular to corresponding ointments.

Semisolid ophthalmic compositions and particularly ointments are one form of a medicament which is suitable for topical administration, for instance to the skin or the eyes. Often, however, it is difficult to incorporate into the ointment an amount of drug sufficient for effectively treating a disorder or disease. Just as well ointment formulations can often not be administered topically to the eye because one or more components of the ointment are only poorly tolerated by the ocular tissue. By the way of example, EP-A-0 474 126 describes ointments comprising the drug ascomycin in an oleagineous ointment base and lower alkylene carbonates or alkane dicarboxylic esters for dissolving an effective amount of the drug in said base. This ointment, however, is not suitable for administration to the eyes.

It has now been found that poly(ethylene-glycols), polyethoxylated castor oils (Cremophor^{®}EL), alcohols having 12 to 20 carbon atoms or a mixture of two or more of said components are valuable excipients for dispersing and/or dissolving effective amounts of ophthalmic drugs, in particular of ascomycins and staurosporine derivatives, in an ointment base, in particular in an ointment base substantially comprising oleaginous and hydrocarbon components, and that the resulting ointments are excellently tolerated by the skin and by ocular tissue.

It has furthermore surprisingly been found that ophthalmic drugs, in particular staurosporine derivatives, comprised in the semisolid compositions according to the invention can easily target the choroid and/or retina in a patient when the compositions are topically administered to the ocular surface, in particular to the sclera of said patient.

A first object of the instant invention is therefore a semisolid ophthalmic composition, in particular an ointment, comprising
- (1): an ophthalmic drug,
- (2): an ointment base and
- (3): an agent for dispersing and/or dissolving said drug in the ointment base, selected from a poly(ethylene-glycol), a polyethoxylated castor oil, an alcohol having 12 to 20 carbon atoms and a mixture of two or more of said components.

Suitable ointment bases include ophthalmically acceptable oil and fat bases, such as
- (a): natural wax e.g. white and yellow bees wax, carnauba wax, wool wax (wool fat), purified lanolin, anhydrous lanolin,
- (b): petroleum wax e.g. hard paraffin, microcrystalline wax,
- (c): hydrocarbons e.g. liquid paraffin, white and yellow soft paraffin, white petrolatum, yellow petrolatum, or
- (d): combinations thereof.

The above mentioned oil and fat bases are described in more detail, for instance, in the British Pharmacopoeia, Edition 2001, or the European Pharmacopoeia, 3^{rd} Edition.

The ointment base may be present in amounts of about 50 to about 95, preferably of 70 to 90% by weight based on the total weight of the composition.

A preferred ointment base comprises a combination of one or more of
- (a): one or more natural waxes like those indicated above, preferably wool wax (wool fat), and
- (c): one or more hydrocarbons like those indicated above, preferably a soft paraffin or a petrolatum, more preferably in combination with liquid paraffin.

A special embodiment of the aforementioned ointment base comprises e. g.
- (a): 5 to 17 parts by weight of wool fat, and
- (c1): 50 to 65 parts by weight of white petrolatum as well as
- (c2): 20 to 30 parts by weight of liquid paraffin.

The agent for dispersing and/or dissolving the ophthalmic drug in the ointment base may be selected from a poly(ethylene-glycol), a polyethoxylated castor oil, an alcohol having 12 to 20 carbon atoms and a mixture of two or more of said components. The agent is preferably used in amounts of 1 to 20 percent, more preferably 1 to 10 percent by weight of the entire semisolid ophthalmic composition.

Alcohols having 12 to 20 carbon atoms include particularly stearyl alcohol (C₁₈H₃₇OH), cetyl alcohol (C₁₆H₃₃OH) and mixtures thereof. Preferred are so-called cetostearyl alcohols, mixtures of solid alcohols substantially consisting of stearyl and cetyl alcohol and preferably comprising not less than 40 percent by weight of stearyl alcohol and a sum of stearyl alcohol and cetyl alcohol amounting to at least 90 percent by weight, and compositions comprising not less than 80 percent by weight of cetylstearyl alcohol and an emulsifier, in particular sodium cetostearyl sulfate and/or sodium lauryl sulfate, preferably in amounts not less than 7 percent by weight of emulsifier.

Polyethoxylated castor oils are reaction products of natural or hydrogenated castor oils and ethylene glycol. Such products may be obtained in known manner, e.g. by reaction of a natural or hydrogenated castor oil or fractions thereof with ethylene oxide, e.g. in a molar ratio of from about 1:30 to about 1:60, with optional removal of free polyethylene glycol components from the product, e.g. in accordance with the methods disclosed in German Auslegeschriften 1,182,388 and 1,518,819. Especially suitable and preferred is a product commercially available under the trade name Cremophor^{®}EL having a molecular weight (by steam osmometry) = ca. 1630, a saponification no.=ca. 65-70, an acid no.=ca. 2, an iodine no.=ca. 28-32 and an n_{D}²⁵ =ca.1.471. Also suitable for use in this category is, for instance, Nikkol^{®}HCO-60, a reaction product of hydrogenated castor oil and ethylene oxide exhibiting the following characteristics: acid no.=ca. 0.3; saponification no.=ca. 47.4; hydroxy value=ca. 42.5. pH (5%)=ca. 4.6; Color APHA=ca. 40; m.p.=ca. 36.0°C.; Freezing point=ca. 32.4°C.; H₂O content (%, KF)=ca. 0.03.

Poly(ethylene-glycols) are the most preferred types of the agent for dispersing and/or dissolving the ophthalmic drug in the ointment base according to the present invention. Suitable poly(ethylene-glycol)s are typically mixtures of polymeric compounds of the general formula H-(OCH₂-CH₂)ₙOH, wherein the index n may typically range from 4 to 230 and the mean molecular weight from about 200 to about 10000. Preferably n is a number from about 6 to about 22 and the mean molecular weight between about 300 and about 1000, more preferably n ranges from about 6 to about 13 and the mean molecular weight from about 300 to about 600, most preferably n has a value of about 8.5 to about 9 and the relative molecular weight is about 400. Suitable poly(ethylene-glycols) are readily available commercially, for example poly(ethylene-glycols) having a mean molecular weight of about 200, 300, 400, 600,1000, 1500, 2000, 3000, 4000, 6000, 8000 and 10000.

The poly(ethylene-glycols), in particular the preferred types described in the foregoing paragraph, are preferably used in amounts of 1 to 10, more preferably 1 to 5 percent by weight of the entire semisolid ophthalmic composition.

An especially preferred embodiment of the compositions according to the instant invention comprises an agent for dispersing and/or dissolving of the drug in the ointment base which is selected from a poly(ethylene-glycol), a polyethoxylated castor oil and preferably a mixture of said components.

Suitable ophthalmic drugs include, for instance, drugs for treatment of the orbit region and ocular appendages, and for treatment of retinal and choroidal diseases, e. g. for the treatment and/or prevention of human retinal and/or chorodial neovascular diseases comprising but not limited to age-related macular degeneration (AMD), diabetic macular edema (DME), proliferative diabetic retinopathy (PDR), for the treatment of glaucoma, inflammation, e.g. blepharitis, and for the treatment bacterial, fungal or viral infections.

The ophthalmic drug is e.g. present in the compositions of this invention in an amount of from about 0.01 to about 10, preferably to 5 percent by weight, e.g. about 0.1 to about 4 percent by weight, e.g. from 0.1 to 2% by weight, e.g. from about 0.3 to about 1% by weight based on the total weight of the composition.

Ophthalmic drugs for the purposes of the present invention include, for example, COX-2 inhibitors of the following formula 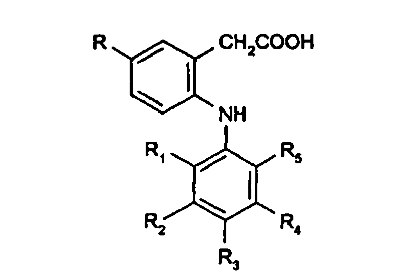 wherein
R is methyl or ethyl;
R₁ is chloro or fluoro;
R₂ is hydrogen or fluoro;
R₃ is hydrogen, fluoro, chloro, methyl, ethyl, methoxy, ethoxy or hydroxy;
R₄ is hydrogen or fluoro; and
R₅ is chloro, fluoro, trifluoromethyl or methyl;
a ophthalmically acceptable salt thereof; or
a ophthalmically acceptable prodrug ester thereof.

Compounds of this type are, for instance, described in US-Patent 6,310,099, and include in particular 5-methyl-2-(2'-chloro-6'-fluoroanilino)phenyl acetic acid.

Ophthalmic drugs for the purposes of the present invention include furthermore e. g. the compounds of the following formula 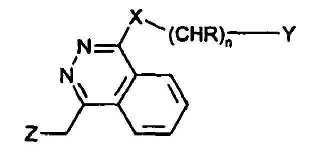 wherein
n is 0 to 2,
R is H or lower alkyl;
X is imino, oxa, or thia;
Y is aryl; and
Z is unsubstituted or substituted pyridyl,
or an N-oxide of the defined compound, wherein one or more N atoms carry an oxygen atom, or an ophthalmically acceptable salt thereof.

Compounds of this type are, for instance, described in US-Patent 6,271,233 and include e.g. 1-(4-Methylanilino)-4-(4-pyridylmethyl)phthalazine; 1-Anilino-4-(4-pyridylmethyl)phthalazine; 1-Benzylamino-4-(4-pyridylmethyl)phthalazine; 1-(4-Methoxyanilino)-4-(4-pyridylmethyl)-phthalazine; 1-(3-Benzyloxyanilino)-4-(4-pyridylmethyl)phthalazine; 1-(3-Methoxyanilino)-4-(4-pyridylmethyl)phthalazine; 1-(2-Methoxyanilino)-4-(4-pyridylmethyl)phthalazine; 1-(4-Trifluoromethylanilino)-4-(4-pyridylmethyl)phthalazine; 1-(4-Fluoroanilino)-4-(4-pyridylmethyl)phthalazine; 1-(3-Hydroxyanilino)-4-(4-pyridylmethyl)phthalazine; 1-(4-Hydroxyanilino)-4-(4-pyridylmethyl)phthalazine; 1-(3-Aminoanilino)-4-(4-pyridylmethyl)-phthalazine; 1-(3,4-Dichloroanilino)-4-(4-pyridylmethyl)phthalazine; 1-(4-Bromoanilino)-4-(4-pyridylmethyl)phthalazine; 1-(3-Chloro-4-methoxyanilino)-4-(4-pyridylmethyl)phthalazine; 1-(4-Cyanoanilino)-4-(4-pyridylmethyl)phthalazine; 1-(3-Chloro-4-fluoroanilino)-4-(4-pyridylmethyl)phthalazine; 1-(3-Methylanilino)-4-(4-pyridylmethyl)phthalazine; and preferably 1-(3-Chloroanilino)-4-(4-pyridylmethyl)phthalazine, as well as ophthalmically acceptable salts of one of said compounds.

Preferred ophthalmic drugs for the purposes of the present invention are ascomycins and, in particular, staurosporine derivatives, especially those according to formula (I) below, or ophthalmically acceptable salts thereof.

Preferred ascomycins for use in the present invention include FK506 or a derivative, antagonist, agonist or analogue of FK506, which retain the basic structure and modulate at least one of the biological properties (for example immunological properties) of FK506 such as described e.g. in EP-A-474 126; 33-epi-chloro-33-desoxy-ascomycin as disclosed in Example 66a in EP-A-427 680 (hereinafter referred to as Compound A); {[1E-(1R,3R,4R)]1R,4S,5R,6S,9R, 10E,13S,15S,16R,17S,19S,20S}-9-ethyl-6,16, 20-trihydroxy-4-[2-(4-hydroxy-3-methoxycyclohexyl)-1-methylvinyl]-15,17-dimethoxy-5,11,13,19-tetramethyl-3-oxa-22-azatricyclo[18.6.1.0(1,22)]heptacos-10-ene-2,8,21,27-tetraone as disclosed in Examples 6d and 71 in EP-A-569 337 (hereinafter referred to as Compound B); and {1R,5Z,9S,12S-[1E-(1R,3R,4R)], 13R,14S,17R,18E, 21S,23S,24R,25S,27R}17-ethyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0(4,9)] octacosa-5,18-diene-2,3,10,16-tetraone, also known as 5,6-dehydroascomycin as disclosed in Example 8 in EP-A-626 385 (hereinafter referred to as Compound C); imidazolylmethyloxyascomycin, as disclosed in Example 1 and as compound of formula I in WO 97/08182 (hereinafter referred to as Compound D); 32-O-(1-hydroxyethylindol-5-yl)ascomycin, also known as Indolyl-ASC or L-732 531 as disclosed in Transplantation 65 (1998) 10-18, 18-26, on page 11, Figure 1 (hereinafter referred to as Compound E); or (32-deoxy-32-epi-N1-tetrazolyl)ascomycin, also known as ABT-281 as disclosed in J. Inv. Derm. 112 (1999), 729-738, on page 730, Figure 1 (hereinafter referred to as Compound F).

FK 506, Compounds A, B, C, D, E, and F are preferred ascomycins, more preferred are Compounds A, B, and C, especially Compound A. Particularly preferred is Compound A.

The instant invention is furthermore particularly useful for staurosporine derivatives as ophthalmic drug, especially for staurosporines of formula (I), wherein R is a hydrocarbyl radical R* or an acyl radical Ac or an ophthalmically acceptable salt thereof.

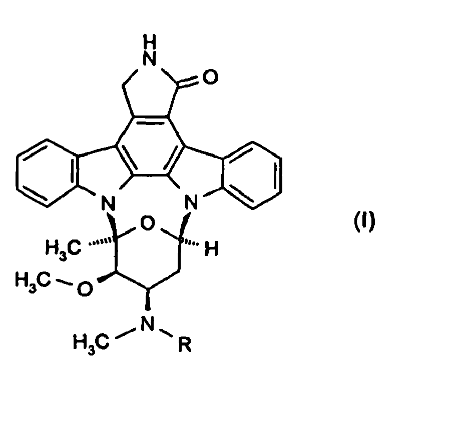

Suitable hydrocarbyl radicals include acyclic, carbocyclic and carbocyclic-acyclic hydrocarbyl radicals having a maximum total number of carbon atoms of preferably 30, especially 18. Additionally suitable hydrocarbyl radicals are heterocyclic radicals and heterocyclic-acyclic radicals. The hydrocarbyl radicals (R^{o}) may be saturated or unsaturated and substituted or unsubstituted. Suitable acyl radicals include optionally functionally modified carboxylic acid and organic sulfonic acid, and optionally esterified phosphoric acid, e.g., pyro- or ortho-phosphoric acid.

Preferred acyclic hydrocarbyl radicals include C₁-C₂₀-alkyl radicals; C₁-C₂₀ hydroxyalkyl radicals of which the hydroxy group is in any position other than the 1-position; cyano-[C₁-C₂₀]-alkyl radicals; carboxy- [C₁-C₂₀]-alkyl radicals of which the carboxy group; and C₃-C₂₀-alkenyl radicals of which the free valency is not at the same carbon atom as the double bond. Exemplary acyclic hydrocarbyl radicals are radicals of lower alkyl, e.g., methyl, ethyl, propyl, butyl, pentyl, hexyl and heptyl; lower alkenyl, e.g., propenyl, 2- or 3-methallyl and 2- or 3-butenyl; lower alkadienyl, e.g., 1-penta-2,4-dinyl; and lower alkynyl, e.g., propargyl or 2-butynyl. Preferred carbocyclic hydrocarbyl radicals are radicals of mono-, bi- or polycyclic cycloalkyl, e.g., cyclopropyl, cyclobutyl, cyclopentyl, cyclohexyl, bicyclo[2,2,2]octyl, 2-bycyclo[2,2,1]heptyl and adamantyl; cycloalkenyl; cycloalkandienyl; and corresponding aryl. Aryl radicals include radicals of phenyl, napthyl (e.g., 1- or 2- napthyl), biphenylyl (e.g., 4-biphenylyl), anthryl, fluorenyl, azulenyl, and aromatic analogues thereof having one or more saturated rings. Preferred carbocyclic-acyclic radicals are acyclic radicals that carry one or more of carbocyclic radicals. Heterocyclic radicals and heterocyclic-acyclic radicals include monocyclic, bicyclic, polycyclic, aza-, thia-, oxa-, thaza-, oxaza-, diaza-, triaza-, and tetraza-cyclic radicals of aromatic character.

Exemplary acyl radicals derived from an optionally functionally modified carboxylic acid (Ac¹) have the formula Z-C(=W)- in which W is oxygen, sulfur, or imino and Z is hydrogen, hydrocarbyl R^{o}, hydrocarbyloxy R^{o}O, or amino. Preferably, W is oxygen or sulfur, and Z is C₁-C₇ alkyl, especially C₁-C₄ alkyl, which is optionally substituted by halogen, carboxy or C₁-C₄ alkoxycarbonyl. Additionally preferred Z is phenyl, pyridyl, furyl, thienyl, imidazolyl, quinolyl, isoquinolyl, benzofuranyl or benzimidazolyl, each of which is unsubstituted or substituted by C₁-C₄ alkyl, C₁-C₄ alkoxy, halogen, nitro, trifluoromethyl, carboxy, C₁-C₄ alkoxycarbonyl, methylenedioxy, cyano and/or a salt thereof. Preferred Ac¹ acyl radicals have the formula R_{b}^{o}-CO-, in which R_{b}^{o} is hydrogen, benzoyl, or a hydrocarbyl radical, e.g., C₁-C₁₉ alkyl radical which is optionally substituted by a carboxy group, cyano group, ester group, amino group or helogen. Another group of preferred Ac¹ acryl radicals have the formula R°-O-CO-. Yet another group of preferred Ac¹ acryl radicals have the formula 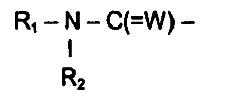 in which R₁ and R₂ are independently selected from hydrogen and unsubstituted acyclic C₁-C₇ hydrocarbyl, preferably C₁-C₄ alkyl and C₃-C₇ alkenyl. R₁ and R₂, independently, can be monocyclic aryl, aralkyl or aralkenyl having a maximum of 10 carbon atoms, each of which is optionally substituted by C₁-C₄ alkyl, C₁-C₄ alkoxy, halogen and/or nitro. Particularly desirable radicals of this group have hydrogen as R₁ and optionally substituted C₁-C₄ alkyl, C₃-C₇ alkenyl, phenyl, pyridyl, pyrimidyl, furyl, thienyl, imidazolyl, quinolyl, isoquinolyl, benzofuranyl or benzimidazolyl as R₂.

Exemplary acyl radicals derived from an organic sulfonic acid (Ac²) have the formula R^{o}-SO₂- in which R^{o} is a hydrocarbyl radical. Preferably, R^{o} of the sulfonic acid acyl radicals is C₁-C₇ alkyl, phenyl, pyridyl, pyrimidyl, furyl, thienyl, imidazolyl, quinolyl, isoquinolyl, benzofuranyl or benzimidazolyl, each of which is unsubstituted or is substituted by C₁-C₄ alkyl, C₁-C₄ alkoxy, halogen, nitro, trifluoromethyl, carboxy, C₁-C₄ alkoxycarbonyl, methylenedioxy, cyano and/or a salt thereof.

Exemplary acyl radicals derived from optionally esterified phosphoric acid (Ac³) have the formula 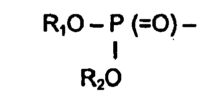 in which R₁ and R₂ are independently selected from hydrogen, unsubstituted acyclic C₁-C₇ hydrocarbyl, preferably C₁-C₄ alkyl and C₃-C₇ alkenyl. R₁ and R₂, independently, can be monocyclic aryl, aralkyl or aralkenyl having a maximum of 10 carbon atoms, each of which is optionally substituted by C₁-C₄ alkyl, C₁-C₄ alkoxy, halogen and/or nitro.

Additionally suitable staurosporine derivatives include staurosporines of formula (I) in which R is derived from an α-amino acid selected from glycine, phenylglycine, alanine, phenylalanine, proline, leucine, serine, valine, tyrosine, arginine, histidine and asparagine, or a salt thereof. Suitable staurosporine derivatives for the present invention are disclosed in further detail in U.S. Pat. No. 5,093,330 to Caravatti et al. The description of the staurosporine derivatives in the patent is herein incorporated by reference.

Particularly preferred staurosporine derivatives of formula (I) for the present invention include: N-(3-carboxypropionyl)-staurosporine, N-benzoyl-staurosporine, N-trifluoracetyl-staurosporine, N-methylaminothiocarbonyl-staurosporine, N-phenylcarbamoyl-staurosporine, N-(3-nitrobenzoyl)-staurosporine, N-(3-fluorobenzoyl)-staurosporine, N-tert-butoxycarbonyl-staurosporine, N-(4-carboxybenzoyl)-staurosporine, N-(3,5-dinitrobenzoyl)-staurosporine, N-alanylstaurosporine, N-ethyl-staurosporine, N-carboxymethyl-staurosporine, N-[(tert.-butoxycarbonylamino)-acetyl]-staurosporine, N-(2-aminoacetyl)-staurosporine, and pharmaceutically acceptable salts thereof.

Especially preferred is N-benzoyl-staurosporine of formula (I), also known in the art as PKC 412 or CGP 41251.

The compositions of the present invention may further comprise a preservative. Suitable preservatives include
• a quaternary ammonium compound such as e.g. benzalkonium chloride (N-benzyl-N-(C₈-C₁₈-alkyl)-N,N-dimethylammonium chloride), benzoxonium chloride, benzethonium chloride, cetrimide (hexadecyl-trimethylammonium bromide), sepazonium chloride, cetylpyridinium chloride, domiphen bromide (Bradosol^{®}) or the like,
• quaternized ammonium cyclodextrin compounds (QACD compounds) having the following formula 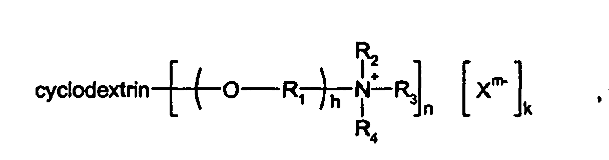 wherein cyclodextrin⁅ ]ₙ
represents a *n*-valent residue derived from a cyclodextrin compound by removing n of its hydroxyl groups;
n is a number greater than 0 and represents the average number of substituents of formula 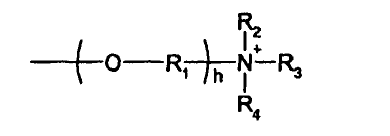 per molecule of said compound;
h is 0 or 1;
R₁ is a di-valent group selected from alkylene, hydroxy alkylene, halogeno alkylene, monocyclic aralkylene, cycloalkylene and phenylene;
R₂, R₃ and R₄ are each independently of one another a group selected from alkyl, cycloalkyl, aryl, aralkyl, and cycloheteryl;
X^{m-} is a m-fold negatively charged anion;
m is an integer being equal or greater than 1; and
k is n/m,
as described, for instance, in U.S.-Patent No. 3,453,257 (index h = 1) or U.S.-Patent 5,241,059 (index h = 0),
• alkyl-mercury salts of thiosalicyclic acid, such as e.g. thiomersal, phenylmercuric nitrate, phenylmercuric acetate or phenylmercuric borate,
• parabens, such as e.g. methylparaben or propylparaben,
• alcohols, such as e.g. chlorobutanol, benzyl alcohol or phenyl ethyl alcohol,
• biguanide derivatives, such as e.g. chlorohexidine or polyhexamethylene biguanide,
• sodium perborate
• imidazolidinyl urea as known and commercially available under the trade name Germal®II,
• sorbic acid,
• stabilized oxychloro complexes such as known and commercially available under the trade name Purite®,
• polyglycol-polyamine condensation resins, such as known and commercially available e.g. under the trade name Polyquart® from Henkel KGaA,
• stabilized hydrogen peroxide generated from a source of hydrogen peroxide for providing an effective trace amount of resultant hydrogen peroxide, e.g. sodium perborate tetrahydrate, and/or
• a mixture of two or more members of such components.

Preferred preservatives are quaternary ammonium compounds, in particular benzalkonium chloride, cetrimide and phenyl ethyl alcohol, a particularly preferred preservative for the purposes of the instant invention. Where appropriate, a sufficient amount of preservative is added to the ophthalmic composition to ensure protection against secondary contaminations during use caused by bacteria and fungi, e.g. benzalkonium chloride and/or cetrimide are present in an amount of about 0.001-0.02%, or phenyl ethyl alcohol is present in an amount of about 0.05 to 5 percent by weight, preferably 0.1 to 2, e. g. 0.1 to about 1 percent by weight.

The compositions of the present invention may further comprise one or more suitable surfactants or emulsifiers, other than wool fat.

The compositions of the present invention may further comprise complexing agents such as
• disodium-ethylenediamine tetraacetate, ethylenediamine tetraacetic acid (EDTA),
• chelating agents having phosphonic acid or phosphonate groups, preferably organophosphonates, particularly amino tri(lower alkylene phosphonic acids) such as those known and commercially available from Monsanto Company, St. Louis, under the trade name Dequest®, or the like,
• cyclodextrins, e.g. α-, β- or γ-cyclodextrin, e.g. alkylated, hydroxyalkylated, carboxy- alkylated or alkyloxycarbonyl-alkylated derivatives, or mono- or diglycosyl-α-, β- or γ- cyclodextrin, mono- or dimaltosyl-α-, β- or γ- cyclodextrin or panosyl-cyclodextrin, e.g. such as known and commercially available under the trade name Cavamax® or Cavasol® from Wacker Chemie, or
• a mixture of two or more of such components.

The compositions of the present invention may further comprise antioxidants such as ascorbic acid, acetylcysteine, cysteine, sodium hydrogen sulfite, butylated hydroxyanisole, butylated hydroxytoluene or natural or synthetic Vitamin E derivatives, such as alpha-tocopherol or alpha-tocopherol acetate.

The compositions of the present invention may further comprise stabilizers such thiourea, thiosorbitol, sodium dioctyl sulfosuccinate or monothioglycerol.

The compositions of the present invention may also comprise a buffer such as acetate, ascorbate, borate, hydrogen carbonate / carbonate, citrate, gluconate, lactate, phosphate, propionate and TRIS (tromethamine) buffers. Tromethamine and borate buffer are preferred buffers. The amount of a buffer added is, for example, that necessary to ensure and maintain a physiologically tolerable pH range. The pH range is typically in the range of from 5 to 9, preferably from 6 to 8.5 and more preferably from 6.5 to 8.2.

It will be appreciated that although the excipients have been described above by reference to a particular function any particular excipient may have alternative or multiple functions, e.g. cyclodextrin or a mixture of cyclodextrins may act as e.g. stabilizer, complexing agent and/or solubilizer.

Information on the properties, specifications and characteristics of the excipients are described e.g. in standard texts such as Fiedler, H.P.; 1996; Lexikon der Hilfsstoffe für Pharmazie, Kosmetik und angrenzende Gebiete; Editio Cantor Verlag Aulendorf (Germany), and Kibbe, A.H.; 2000; Handbook of Pharmaceutical Excipients, a joint publication of Pharmaceutical Press, London (UK), and American Pharmaceutical Association, Washington (US) as well as manufacturers' brochures, the contents of which are incorporated herein by reference.

The compositions of the present invention may further comprise water in an amount of less than 10% by weight based on the total amount of composition.

Preferably, the compositions of the present invention are free of components such as perfumes or colorants.

A preferred embodiment of the compositions according to the present invention comprises or, preferably, consists essentially of
- (1): an ophthalmic drug, in particular a staurosporine derivative of formula (I) above, more particular PKC 412,
- (2): an ointment base, preferably comprising a combination of

- (a): one or more natural waxes, preferably a wool wax (wool fat), and
- (c): one or more hydrocarbons, preferably a soft paraffin or a petrolatum, more preferably in combination with a liquid paraffin,

- (3): an agent for dispersing and/or dissolving the drug in the ointment base selected from a poly(ethylene-glycol) and a mixture of a poly(ethylene-glycol) and a polyethoxylated castor oil, and optionally
- (4): a preservative, in particular phenyl ethyl alcohol and, optionally too,
- (5): an antioxidant, in particular a natural or synthetic Vitamin E derivative.

Especially preferred is a composition according to this invention comprising or, more preferably, essentially consisting of
- (1): 0.1 to 4, preferably 0.4 to 1.5 percent of a staurosporine derivative of formula (I), particularly PKC 412;
- (2): an ointment base, essentially consisting of a combination of

- (a): 5 to 17 percent of wool fat,
- (c1): 50 to 65 percent of white petrolatum and
- (c2): 20 to 35 percent of liquid paraffin;

- (3): 1 to 10 percent of an agent for dispersing and/or dissolving the drug in the ointment base essentially consisting of a poly(ethylene-glycol), optionally in combination with a polyethoxylated castor oil, and
- (4): 0.01 to 2 percent of a preservative, in particular phenyl ethyl alcohol as well as
- (5): 0.01 to 2 percent of an antioxidant, in particular a natural or synthetic Vitamin E derivative,
wherein all percentages relate to the total weight of the components (1) to (5) and only combinations of percentages are permitted which add up to 100.

The ophthalmic drug may, for instance, be dispersed or dissolved or partially dispersed and partially dissolved in the vehicle. So, the ophthalmic drug may, for instance, be in suspension or partially in suspension in the vehicle. Preferably, however, the ophthalmic drug is dissolved in the vehicle, at least partially.

If the drug is suspended in the vehicle, the drug is preferably used in a micronized form. Such suspensions may contain particles of a solid ophthalmic drug, e.g. of a staurosporine derivative or an ascomycin, of from 5, e.g. from 10, to about 90, preferably to about 25 microns in diameter. Such drug particles may be produced in conventional manner, e.g. by grinding or milling. In case the active agent exists in different polymorphic or pseudo-polymorphic forms, the thermodynamic stable form is preferably used in a suspension type formulation.

The compositions of the present invention are stable, as indicated by conventional tests, e.g. under stressed conditions, such as a temperature cycling test at 5 to 30°C, for several months, e.g. 1 to 12 months, at 30°C.

The ophthalmic compositions of the present invention may be prepared in conventional manner e.g. by mixing the preferably gamma-irradiated ophthalmic drug, e.g. the staurosporine derivative or ascomycin with the poly(ethylene-glycol) and/or the polyethoxylated castor oil and/or an alcohol having 12 to 20 carbon atoms, if necessary under heating and/or sonication, e. g. for 0.5 to 6 hours, optionally adding further excipients, such as e. g. a preservative and/or an antioxidant, and mixing the resulting composition with the melted ointment base, sterilizing the mixture, for instance by filtration through a 0.2 micron filter, and cooling it thereafter to room temperature under stirring. It is of course also possible to sterilize the molten ointment base and the partial composition comprising the drug separately before combining them to form the ointment.

The semisolid compositions, in particular ointments, according to the present invention are particularly useful for topical administration of ophthalmic drugs to the skin, e. g. the skin of the eyelid, or in the cul du sac of the eye or to the surface of the eye, in particular in particular to the cornea, sclera and/or conjunctiva.

Compositions according to the invention comprising staurosporine derivatives like those mentioned beforehand are particularly useful in the treatment and/or prevention of human ocular neovascular diseases, more particularly in the treatment and/or prevention of human retinal and/or chorodial neovascular diseases. Indications of particular interest for the use of the semisolid compositions or ointments according to the instant invention comprising a staurosporine derivative are the treatment and/or prevention of age-related macular degeneration (AMD), diabetic macular edema (DME), proliferative diabetic retinopathy (PDR).

In another aspect the present invention therefore provides a composition as defined above and comprising a staurosporine derivative as mentioned above for use in the treatment and/or prevention of human ocular neovascular diseases, more particularly in the treatment and/or prevention of human retinal and/or choroidal neovascular diseases, most preferably in the treatment and/or prevention of age-related macular degeneration (AMD), diabetic macular edema (DME), and/or proliferative diabetic retinopathy (PDR).

In another aspect the present invention provides a method for the treatment and/or prevention of human ocular neovascular diseases, more particularly the treatment and/or prevention of human retinal and/or chorodial neovascular diseases, most preferably the treatment and/or prevention of age-related macular degeneration (AMD), diabetic macular edema (DME), and/or proliferative diabetic retinopathy (PDR), comprising topically administering a composition as defined above and comprising a staurosporine derivative to the ocular surface, e. g. the sclera of a patient in need thereof.

More specifically said method comprises the topical administration of the staurosporine derivative comprised in the semisolid compositions according to the invention to the ocular surface, in particular to the sclera of the patient, thereby targeting the choroid and/or retina in said patient.

In another aspect the present invention provides the use of a composition as defined above and comprising a staurosporine derivative in the preparation of a medicament for topically administering said staurosporine derivative to the ocular surfaces of the eye, e.g. to the sclera, of a patient in need thereof, thereby targeting the choroid and/or retina in said patient.

In yet another aspect the present invention provides the use of a composition as defined above and comprising a staurosporine derivative in the preparation of a medicament for the treatment and/or prevention of human ocular neovascular diseases, more particularly for the treatment and/or prevention of human retinal and/or chorodial neovascular diseases, most preferably for the treatment and/or prevention of age-related macular degeneration (AMD), diabetic macular edema (DME), and/or proliferative diabetic retinopathy (PDR) in a patient in need of such treatment.

The semisolid compositions according to the instant invention comprising an ascomycin, in particular corresponding ointments, have also a variety of pharmacological actions and are useful e.g. in the treatment of of inflammatory diseases, especially blepharitis e.g. chronic blepharitis, e.g. seborrhoeic blepharitis or allergic blepharitis.

In another aspect the present invention therefore provides a composition as defined above and comprising an ascomycin for use in the treatment of inflammatory diseases, especially blepharitis e.g. chronic blepharitis, e.g. seborrhoeic blepharitis or allergic blepharitis.

In another aspect the present invention provides a method for treating inflammatory diseases, especially blepharitis, e.g. chronic blepharitis, e.g. seborrhoeic blepharitis or allergic blepharitis, comprising topically administering a composition as defined above and comprising an ascomycin to the skin of a patient in need thereof.

In another aspect the present invention provides the use of a composition as defined above and comprising an ascomycin in the preparation of a medicament for topically administering to the eye, e.g. on the skin of the eyelid or in the cul de sac of the eye or upon the ocular surfaces of the eye, of a patient in need thereof.

In yet another aspect the present invention provides the use of a composition as defined above and comprising an ascomycin in the preparation of a medicament for the treatment of inflammatory diseases, especially blepharitis e.g. chronic blepharitis, e.g. seborrhoeic blepharitis or allergic blepharitis.

The utility of the compositions according to the invention can be observed in standard clinical tests such as the test set out below.

One animal test comprises a modified Draize test on three albino rabbits wherein the ocular tolerability after a single dose instillation of 50 microlitres of compositions of the present invention on eyelid or the ocular surface is shown for the 15 minutes after application then after 1, 2 and 7 days. The tolerability was based on visual examination considering the following parameters: discomfort as judged by blinking or partial/complete closure of the eye, duration of discomfort, discharge, redness of conjunctiva (palpebral and bulbar conjunctiva), chemosis of conjunctiva (swelling), degree of opacity of cornea and area of cornea involved, and pathological influence upon iris.

The compositions of the invention are found to be effective and well tolerated.

The exact amount of the ophthalmic drug and of the composition to be administered depends on several factors, for example the targeted disease, the species, age, weight and physical condition of the subject, desired duration of treatment and pharmacological data, e. g. the rate of release of the drug. Satisfactory results are obtained in larger mammals, e.g. humans, with the local application upon the ocular tissue to be treated or upon the ocular surfaces of the eye of a 0.01 to 5% by weight concentration of the drug once or several times a day, in particular if an ascomycin or staurosporine derivative is administered.

The following Examples illustrate the invention.

### Example 1

A typical unpreserved ointment according to the invention may look like

| Ingredient | Example 1¹⁾ |
|---|---|
| CGP 41251²⁾ | 1.00 |
| Wool fat | 5.52 |
| White petrolatum | 60.52 |
| Liquid paraffin | 26.96 |
| PEG 400³⁾ | 6.00 |
| | |
| Total | 100 |

| | |
|---|---|
| ¹⁾ amounts in percent weight/weight (%w/w) ²⁾ staurosporine derivative of formula (I), wherein R is a benzoyl residue ³⁾ poly(ethylene-glycol) of H-(OCH₂CH₂)ₙOH, wherein the index n has a value of about 8.5 to about 9 (mean relative molecular weight about 400). | |

The preparation may be carried out as follows:

The staurosporine derivative is dissolved in the poly(ethylene-glycol) under heating and sonication and this solution is thoroughly mixed with the molten ointment base comprising the wool fat, the white petrolatum and the liquid paraffin. The resulting liquid composition is then stirred until it reaches room temperature.

### Example 2 to 7 (preserved ointments)

| Ingredient | Example 1 % w/w | Example 2 % w/w | Example 3 % w/w |
|---|---|---|---|
| PKC 412 | 0.5 | 0.75 | 1.0 |
| White petrolatum | 60 | 60 | 60 |
| Wool fat | 6 | 6 | 6 |
| Liquid paraffin | 29.9 | 26.25 | 25.5 |
| PEG 400 | 3 | 6 | 6 |
| Phenylethyl alcohol* | 0.5 | 0.5 | 0.5 |
| Alpha-tocopherol | 0.1 | 0.5 | 1.0 |

| Ingredient | Example 4 % w/w | Example 5 %w/w | Example 6 % w/w |
|---|---|---|---|
| PKC 412 | 0.5 | 0.75 | 1.0 |
| White petrolatum | 60 | 60 | 60 |
| Wool fat | 6 | 6 | 6 |
| Liquid paraffin | 29.9 | 26.25 | 25.5 |
| PEG 4000 | 3 | 6 | 6 |
| Phenylethyl alcohol* | 0.5 | 0.5 | 0.5 |
| Alpha-tocopherol | 0.1 | 0.5 | 1.0 |

| | | | |
|---|---|---|---|
| *Phenylethyl alcohol can readily be replaced, for instance, with 0.01% of benzalkonium chloride or cetrimide. | | | |

### Manufacturing Procedure

- 1.: Weigh liquid paraffin in a media bottle. Remove air bubbles in the liquid paraffin by pulling a vacuum on it for a period of 30 minutes or until all air bubbles are removed.
- 2.: Melt white petrolatum and wool fat on a stir plate at approximately 70°C.
- 3.: Mix gently the liquid paraffin, white petrolatum and wool fat at approximately 70°C for a period of 15 minutes or until the ingredients are uniformly mixed to form Part 1.
- 4.: Dissolve PKC 412 in PEG 400 or PEG 4000 by sonicating PKC 412 in a water bath maintained at 45°C. It takes approximately 4 hrs to completely dissolve PKC 412. This is Part 2.
- 5.: Add alpha-tocopherol and phenylethyl alcohol or benzalkonium chloride or cetrimide to the PEG containing PKC 412 (Part 2). This is Part 3.
- 6.: Add dissolved PKC 412 (Part 3) to Part 1 and mix for a period of 45 minutes or until a uniform dispersion is obtained.
- 7.: The PKC 412 ointment is now ready for sterilization.
- 8.: The PKC 412 ointment can be sterilized by filtrating the molten ointment through a 0.2 micron filter.
- 9.: Alternatively, the filtration sterilization process can be achieved by filtering Part 1 through a 0.2 micron filter, followed by filtering Part 2 through a 0.2 micron filter and subsequently combine the sterile Part 1 and Part 2 to form a sterile PKC 412 ointment.

## Claims

1. Semisolid ophthalmic composition, in particular ointment, comprising
(1) an ophthalmic drug,
(2) an ointment base and
(3) an agent for dispersing and/or dissolving said drug in the ointment base, selected from a poly(ethylene-glycol), a polyethoxylated castor oil, an alcohol having 12 to 20 carbon atoms and a mixture of two or more of said components.

2. Composition according to claim 1, wherein the ointment base comprises
(a) a natural wax, preferably a white or yellow bees wax, a carnauba wax, a wool wax (wool fat), a purified lanolin or an anhydrous lanolin.
(b) a petroleum wax, preferably a hard paraffin or a microcrystalline wax,
(c) a hydrocarbon, preferably a liquid paraffin, a white or yellow soft paraffin or a white petrolatum or yellow petrolatum, or
(d) a combination of two or more of such components.

3. Composition according to claim 1, wherein the agent for dispersing and/or dissolving the drug in the ointment base is selected from a poly(ethylene-glycol), a polyethoxylated castor oil and a mixture of said components.

4. Composition according to claim 3, wherein the agent for dispersing and/or dissolving the drug in the ointment base is a mixture of a poly(ethylene-glycol) and a polyethoxylated castor oil.

5. Composition according to claim 3 or 4, wherein the poly(ethylene-glycol) has the formula H-(OCH₂-CH₂)ₙOH, wherein n is a number from about 6 to about 22.

6. Composition according to claim 3 or 4, wherein the polyethoxylated castor oil is Cremophor®EL.

7. Composition according to claim 1, wherein the agent for dispersing and/or dissolving the ophthalmic drug in the ointment base is used in amounts of 1 to 20 percent by weight of said composition.

8. Composition according to claim 1, wherein the ophthalmic drug is selected from
• a COX-2 inhibitor of the following formula 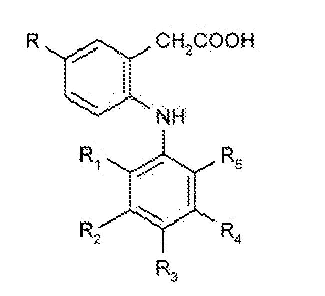 wherein
R is methyl or ethyl;
R₁ is chloro or fluoro;
R₂ is hydrogen or fluoro;
R₃ is hydrogen, fluoro, chloro, methyl, ethyl, methoxy, ethoxy or hydroxy:
R₄ is hydrogen or fluoro; and
R₅ is chloro, fluoro, trifluoromethyl or methyl,
preferably 5-methyl-2-(2'-chloro-6'-fluoroanilino)phenyl acetic acid,
an ophthalmically acceptable salt thereof; or
an ophthalmically acceptable prodrug ester thereof;
• a compound of the following formula 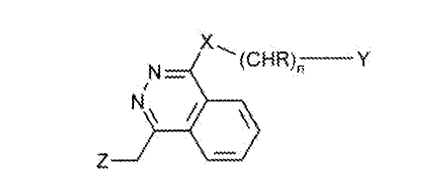 wherein
n is 0 to 2,
R is H or lower alkyl;
X is imino, oxa, or thia;
Y is aryl; and
Z is unsubstituted or substituted pyridyl,
or an N-oxide of the defined compound, wherein one or more N atoms carry an oxygen atom,
preferably 1-(3-Chloroanilino)-4-(4-pyridylmethyl)phthalazine,
or an ophthalmically acceptable salt thereof; and particularly from
• an ascomycin;
• a staurosporine derivative, especially a staurosporine derivatives of formula (I) 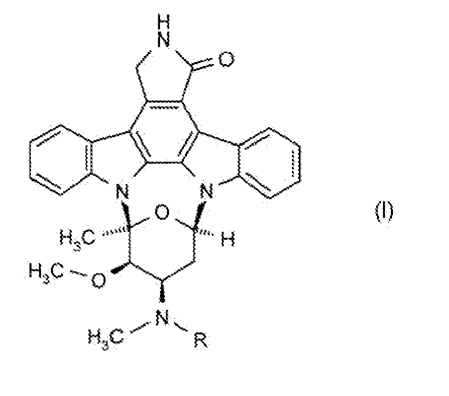 wherein
R is a hydrocarbyl radical or an acyl radical, or an ophthalmically acceptable salt thereof.

9. Composition according to claim 8, wherein the ophthalmic drug is a staurosporine derivative of formula (I) and R is preferably benzoyl (also known as PKC 412 or CGP 41251).

10. Composition according to claim 1 or 8, comprising or, preferably, consisting essentially of
(1) an ophthalmic drug, in particular a staurosporine derivative of formula (I),
(2) an ointment base, preferably comprising a combination of
(a) one or more natural waxes, preferably a wool wax (wool fat), and
(b) one or more hydrocarbons, preferably a soft paraffin or a petrolatum, more preferably in combination with a liquid paraffin,
(3) an agent for dispersing and/or dissolving the drug in the ointment base selected from a poly(ethylene-glycol) and a mixture of a poly(ethylene-glycol) and a polyethoxylated castor oil, and optionally
(4) a preservative, in particular phenyl ethyl alcohol and, optionally too,
(5) an antioxidant, in particular a natural or synthetic Vitamin E derivative.

11. Composition according to claim 8 comprising or, preferably, essentially consisting of
(1) 0.1 to 4, preferably 0.4 to 1.5 percent of a staurosporine derivative of formula (I), more particular PKC 412;
(2) an ointment base, essentially consisting of a combination of
(a) 5 to 17 percent of wool fat,
(c1) 50 to 65 percent of white petrolatum and
(c2) 20 to 35 percent of liquid paraffin;
(3) 1 to 10 percent of an agent for dispersing and/or dissolving the drug in the ointment base essentially consisting of a poly(ethylene-glycol), optionally in combination with a polyethoxylated castor oil, and
(4) 0.01 to 2 percent of a preservative, in particular phenyl ethyl alcohol as well as
(5) 0.01 to 2 percent of an antioxidant, in particular a natural or synthetic Vitamin E derivative.
wherein all percentages relate to the total weight of the components (1) to (5).

12. Composition according to claim 8 and comprising a staurosporine derivative for use in the treatment and/or prevention of human ocular neovascular diseases, more particularly in the treatment and/or prevention of human retinal and/or chorodial neovascular diseases, most preferably in the treatment and/or prevention of age-related macular degeneration (AMD), diabetic macular edema (DME), and/or proliferative diabetic retinopathy (PDR).

13. Composition according to claim 12 wherein the staurosporine derivative comprised in said semisolid composition is administered topically to the ocular surface, in particular to the sclera of the patient, thereby targeting the choroid and/or retina in said patient.

14. Composition according to claim 8 and comprising an ascomycin for use in the treatment of inflammatory diseases, especially blepharitis e.g. chronic blepharitis, e.g. seborrhoeic blepharitis or allergic blepharitis.

15. Composition according to claim 8, wherein said composition is adapted to topical administration to the eye and/or its environment, e.g. to the skin of the eyelid or into the cul de sac of the eye or upon the ocular surfaces of the eye, of a patient in need thereof.
